# EUROPEAN PATENT APPLICATION

(11) **EP 4 624 928 A1**
(43) Date of publication of application: **01.10.2025**
(21) Application number: 24382338.2
(22) Date of filing: 27.03.2024
(51) Int. Cl.: G01N 33/84

(54) **MATERIAL CAPABLE OF SENSING REACTIVE OXYGEN SPECIES IN A LIQUID MEDIA**

(71) Applicant: Arrays for Cell Nanodevices S.L., 28223 Pozuelo de Alarcón Madrid (ES)
(72) Inventor: PALOMARES ALBARRÁN, María, 28223 Pozuelo de Alarcón (ES); PALACIOS DOIZTÚA, Irene, 28223 Pozuelo de Alarcón (ES); MONTALVILLO JIMÉNEZ, Laura, 28223 Pozuelo de Alarcón (ES); GARCÍA SORIANO, David, 28223 Pozuelo de Alarcón (ES); MARTÍNEZ BARTOLOMÉ, Marina, 28223 Pozuelo de Alarcón (ES); MIGUEZ LABANDEIRA, Rubén, 28223 Pozuelo de Alarcón (ES); HERNÁNDEZ PINTO, Alberto Miguel, 28223 Pozuelo de Alarcón (ES); SÁNCHEZ SANCHO, Francisco, 28223 Pozuelo de Alarcón (ES)
(74) Representative: Hoffmann Eitle

(57) **Abstract**

The present invention refers to the chemical field. Particularly, it refers to a method for obtaining a material capable of sensing reactive oxygen species (ROS) in a liquid media, wherein the method comprises: a) providing a material comprising a surface activated with hydroxyl groups, b) carrying out a silanization process of the hydroxyl groups of step a) with an organofunctional alkylalkoxysilane molecule, and c) reacting the silanized material resulting from step b) with a ROS sensor, wherein said reaction is under conditions suitable for the formation of a covalent bond between the ROS sensor and the organofunctional alkylalkoxysilane molecule. It also refers to materials capable of sensing ROS in a liquid media, and to their *in vitro* use in a method for the determination of ROS in a liquid media.

## Description

### TECHNICAL FIELD

The present invention refers to the chemical field. Particularly, it refers to materials capable of sensing reactive oxygen species (ROS) in a liquid media, and to their *in vitro* use in the determination of ROS in a liquid media, preferably intracellularly.

### BACKGROUND ART

Energy metabolism is the process by which cells obtain energy in the form of adenosine triphosphate (ATP) from nutrients. This process encompasses a series of interconnected pathways that function in the absence or presence of molecular oxygen (anaerobic or aerobic metabolism, respectively) that must be maintained in balance.

In eukaryotic cells, the core of these pathways occurs in the presence of oxygen in the mitochondria. Thanks to them, the cell obtains a large amount of energy in the form of ATP (30-32 moles for each mole of glucose) and reducing power in the form of NADH and FADH, releasing CO2 and water. Although mitochondrial respiratory complexes are efficient, their normal physiological activity generates a series of unstable byproducts, called reactive oxygen species (ROS), which have the capacity to alter the function of biomolecules such as proteins, lipids and nucleic acids. Therefore, they must be maintained at basal levels and are highly controlled by a series of antioxidant defenses that transform them into stable and harmless molecules for the cell. An imbalance between ROS production and its elimination has been described in many cellular disorders, a condition known as cellular stress and which can arise from a decrease in the effectiveness of antioxidant defenses, an increase in ROS production (often caused due to mitochondrial dysfunction), or both.

As a fundamental pillar of cellular metabolism, mitochondrial disorders underlie the bases of numerous disorders and pathologies such as inflammation, premature aging, cancer, neurodegeneration or diabetes.

In light of this background, it is not surprising, therefore, that research into mitochondrial health and energy metabolism has gained much interest in the study of diseases, discovery of new drugs and personalized medicine. Therefore, the development of new tools for a better characterization of these cellular phenomena is a necessity.

The most classic strategies to analyse certain cellular components have been fractionation and subsequent purification or fixing the cells in a specific state of their cycle for subsequent characterization. However, these strategies do not always allow us to determine each component of a complex system separately, analyse each cell individually, or know how a cell interacts with its microenvironment. After decades of research in this area, cell biology is increasingly moving away from the study of cell populations to focus on single-cell studies. Population analyses are based on the premise that the average response of a population is similar to, or at least representative of, the behaviour of individual cells. However, although this premise may be true, it is increasingly clear that heterogeneity is a fundamental attribute of living organisms and, therefore, the cells of a population can differ epigenetically, transcriptomics and biochemically. This means that, although at the population level the response to a stimulus may seem homogeneous and gradual, there are differences at the individual level that current techniques are not always able to detect due to their limitations. Therefore, to better understand cellular biochemistry and pathophysiological processes, it is necessary to develop new technologies that allow real-time monitoring of the individual responses that different cells of the same tissue or population present to a certain stimulus.

In recent years, the development of "omics" technologies has gained great importance due to the high degree of information they are capable of offering about a cell population, both at the population and single cell level. Unfortunately, in the study of cellular and mitochondrial metabolism, metabolomics techniques have not yet reached sufficient sensitivity to work at the single cell level nor do they allow information to be obtained from living cells (they are endpoint techniques). Therefore, to analyse these parameters completely, a combination of complementary approaches is usually necessary. For example, at the single cell level, chemical probes, capable of passing through the membrane, are often used, which can be analysed by flow cytometry or image analysis platforms, whether conventional microscopes, confocal or high-throughput coelomic systems (HCS). However, these probes, being soluble, interfere with cellular metabolism or are toxic, which can generate erroneous readings and limit analysis time.

Microelectromechanical systems (MEMS) are small silicon microdevices able to operate at the nanoscale by combining the knowledge of physics, mechanics and chemistry into a single device. Recent advances in this ground-breaking field allow the manufacture at a subcellular scale of micromachines and biosensors with functional parts. Thus, MEMS are gaining importance in experimental biomedicine being used for monitoring body physiology, as tissue scaffolding devices or systems for controlled drug delivery. Among others, MEMS have been used to develop precise delivery experimental systems based in microneedles, able to inject exact amount of therapeutics in specific skin locations. Besides those extracellular applications, in the last years it has been also demonstrated that living cells incorporate MEMS into the cytosol without significant loss of their viability.

ROS are commonly used to define the reactive molecules and free radicals originating from molecular oxygen. These oxidants include molecules that are free radicals and non-radicals. A free radical contains an unpaired electron in its outer orbital, which renders it highly reactive and indiscriminate in its reactions. Free radicals derived from oxygen are the superoxide anion (O2•⁻) and its protonated form, the perhydroxylradical (HO₂•), the hydroxyl radical (HO•), the peroxyl radical (ROO•) and the alkoxyl radical (RO•) (R is a lipid or protein). Non-radical ROS include hydrogen peroxide (H₂O₂), singlet molecular oxygen ('Oz), hypochlorous acid (HOCI), and organic hydroperoxides (ROOH). A large number of studies have proved the messenger role of ROS in cell survival, proliferation, differentiation, death and apoptosis. Different levels of ROS have been proved to play distinct roles in cell life: low concentrations of ROS could stimulate the mitosis and proliferation of cells, while a moderate dose would inhibit cell cycle, and with increase of their concentration, apoptosis might be activated. Moreover, different types of oxygen radicals behave differently in cells, depending on their respective characteristics in reaction preferences, site of production, degradation and diffusion. Consequently, in order to figure out the biological impacts of ROS, it is critical to know their accurate locations and concentrations. However, due to the instability, short life and mutual interference of most oxygen radicals, it is not easy to achieve all the above objectives. ROS were previously thought to emerge almost exclusively from mitochondrial metabolism. Nevertheless, there is growing evidence that cellular enzymes known as nicotinamide adenine dinucleotide phosphate (NADPH) oxidases produce a considerable amount of ROS in humans. Other cellular sources of ROS include neutrophils, monocytes, cardiomyocytes, endothelial cells, xanthine oxidases, cytochrome P450, lipoxygenases, and nitric oxide synthases. In physiological systems, ROS generation exists in concert with a wide variety of antioxidant defenses like superoxide dismutase (SOD), catalase, peroxiredoxins (Prx), glutathione peroxidases (GPx), glutathione, vitamin C and vitamin E. The redox equilibrium safeguards cells against higher, toxic levels of ROS. Due to their high reactivity, higher doses of ROS (oxidants) can cause non-specific damage to macromolecular targets such as proteins, enzymes, lipids, membranes, and nucleic acids, and ultimately cell death by apoptosis and/or necrosis. The inequality between the systemic production of ROS and the ability of cells to instantly detoxify the reactive intermediates or to restore the resultant impairment is often called as "oxidative stress" (OS). Oxidative stress is defined as "a serious imbalance between the generation of ROS and antioxidant defenses in favor of ROS, causing excessive oxidative damage to biomolecules". Recently, OS has been associated with a broad range of degenerative processes, diseases, and syndromes. Conversely, ROS are not always considered as harmful metabolic byproducts; they play the role of intracellular signalling molecules when strictly regulated. In addition, at cellular levels, ROS contribute to complicated functions, such as blood pressure regulation, cognitive functions, and immune responses.

Detoxification of ROS is fundamental for all cells to survive. Living organisms have evolved a variety of defense mechanisms to provide a balance between generation and elimination of ROS to endure the oxygen-rich cellular environment.

Despite the difficulties in measuring ROS, many types of methods are available, like the use of fluorescent and chemiluminescent probes, spectrophotometry, and spectrometry methods, as well as chromatography and genetically encoded fluorescent protein-based assays. The use of fluorescence has become commonplace in the biological sciences, with many studies utilizing probes based on diverse fluorophores to provide insight into cell function and behaviour. As these imaging applications become more advanced it becomes increasingly important to acquire accurate quantitative measurements of the fluorescence signal. Because of their involvement in a myriad of diseases, ROS probes for both *in vivo* and *in vitro* detection are vital tools for clinical diagnostics. Although many probes have been developed, several problems need to be addressed to successfully yield a multipotent ROS probe. Some of the considerations in designing in situ ROS fluorescent probes include water solubility, aggregation, quantum yields, singlet excited-state lifetime, and excitation wavelength, which determines tissue penetration. Additionally, biologic considerations such as cell permeability, ROS selectivity, and reaction rates are important for meaningful detection. Because of the complex cellular environments in which ROS are monitored, numerous diverse probes have been developed. A common strategy to trigger a fluorescent readout in response to ROS is the use of a nonfluorescent reduced dye that, when oxidized by ROS, yields a fluorescent product. Reduced dyes are particularly attractive for ROS detection because they often can be generated from commercially available fluorescent dyes, allowing for trivial synthesis of a wide range of reduced dyes that can be used to detect and image ROS.

Xanthene dyes are some of the most frequently used probes for ROS detection, with reduced derivatives of fluorescein and rhodamine being the most abundant. They can easily be prepared through direct reduction of their oxidized derivatives. Xanthene dyes have been shown to react readily with hydroxyl and peroxyl radicals and with several reactive nitrogen species (RNS). As a consequence, fluorescein and rhodamine-based dyes have been heavily used for ROS detection. Absolute quantification of fluorescence, however, requires the fluorophores themselves to be insensitive to environmental factors such as non-specific protein interactions and pH. It is widely known that the fluorescent intensity of fluorescein is highly susceptible to changes in pH and other environmental factors.

Similarly, hydrocyanines have been extensively investigated for ROS imaging. They can be easily obtained by reducing commercially available cyanine dyes with NaBH₄. Hydrocyanine probes have been used for detection of superoxide and hydroxyl ROS both *in vitro* and *in vivo,* with detection limits down to nanomolar concentrations. A wide range of hydrocyanines with desired properties can be prepared in a single synthetic step, making them accessible even to labs with limited synthetic infrastructures. Although hydrocyanines have many uses, they do have some limiting factors. These limitations include high autooxidation, low Stokes shifts, and low solubility, as well as their product cyanine dyes' lability to ROS.

Many other probes have been developed in recent years employing different strategies for ROS sensing. However, most of them share some limitations:
- High cytotoxicity, compromise cell viability and interference in the cell homeostasis, especially when maintaining cell cultures for long periods of time (>24 hours);
- Poor tissue penetration as it is not always possible to limit the presence of the probe in the cytoplasm;
- Limited usage time inside the cell (until probes are metabolized or excreted);
- Reliance on end-point assays that measure a single analysis parameter that might bias the interpretation of the analyses, increasing the sample requirement.

All these issues combined limit the information that can be collected when carrying out scientific research. Despite the significant leap forward in the cell imaging equipment, no relevant technological developments in fluorescents probes and reagents have been introduced in the market. Those reagents have lost track of the innovations occurred in the field in the last years, resulting in tools with lack of reproducibility and translatability to clinical trials, thus, further technological developments are required.

The present invention provides a method for obtaining a material capable of sensing ROS in a liquid media, as well as a material capable of sensing ROS in a liquid media.

### DESCRIPTION OF THE FIGURES

**Figure 1****.** ROS SPAchip manufacture
**Figure 2****.** Synthetic route to Compound 1 required for the functionalization SPAchip.
**Figure 3****.** SPAchip functionalization with Compound 1 (ROS SPAchip)
**Figure 4****.** Selectivity of ROS SPAchips at 90 minutes (light grey) and at 24 hours (dark grey).
**Figure 5****.** No significant effect of pH on the fluorescence of DCF SPAchips.
**Figure 6****.** Fibroblast 1095sk cell line with the DCF SPAchip. Image acquired with 40x magnification objective. In blue, nuclear staining by Hoechst 3342, in red, cytoplasmatic staining by Cell Mask^{™} Plasma Membrane and the intracellular and extracellular SPAchip in green.
**Figure 7****.** Fluorescence corrected intensity of ROS detection probe free in solution (a) and coupled to SPAchips (b) within Fibroblast 1095sk cell line after 6 hours of treatment with different drugs concentrations: Doxorubicin (DOXO) and Tert-Butyl hydroperoxide (TBHP).

### SUMMARY OF THE INVENTION

A first aspect of the invention refers to a method for obtaining a material capable of sensing ROS in a liquid media, wherein the method comprises:
a) providing a material comprising a surface activated with hydroxyl groups,
b) carrying out a silanization process of the hydroxyl groups with an organofunctional alkylalkoxysilane molecule, selected from the group consisting of aminoalkylalkoxysilane, mercaptoalkylalkoxysilane and hydroxyalkylalkoxysilane, and
c) reacting the silanized material resulting from step b) with a ROS sensor, wherein said reaction is under conditions suitable for the formation of a covalent bond between the ROS sensor and the organofunctional alkylalkoxysilane molecule.

In a preferred embodiment the ROS sensor is 2',7'-dichlorodihydrofluorescein diacetate, or a salt, solvate, stereoisomer or ester therefrom.

In another preferred embodiment step c) comprises reacting the silanized material resulting from step b) with the ROS sensor 2',7'-dichlorodihydrofluorescein diacetate, wherein the organofunctional alkylalkoxysilane molecule is an organofunctional aminoalkylalkoxysilane molecule, wherein said reaction is under conditions suitable for the formation of an amide bond between the carboxylic group present at the ROS sensor 2',7'-dichlorodihydrofluorescein diacetate, and an amine group present at the organofunctional aminoalkylalkoxysilane molecule.

In another preferred embodiment the organofunctional aminoalkylalkoxysilane molecule is selected from the group consisting of 3-aminopropyltriethoxysilane (APTES), 3-aminopropylmethyldiethoxysilane (APMDES), 3-aminopropyldimethylethoxysilane (APDMES), (3-aminopropil)trimetoxisilano (APTMS) and aminoethylaminopropyltriethoxysilane.

In another preferred embodiment the organofunctional aminoalkylalkoxysilane molecule is APTES.

In another preferred embodiment the ROS sensor is 2',7'-dichlorodihydrofluorescein diacetate, or a salt, solvate, stereoisomer or ester therefrom, wherein the organofunctional aminoalkylalkoxysilane molecule is APTES.

In another preferred embodiment the surface of the material is made of silicon oxide, preferably the material is made of silicon oxide. Preferably, the silicon oxide is silicon dioxide. Other potentially useful materials include any silicon derivative, such as silicon oxide, polysilicon, monocrystalline silicon.

In another preferred embodiment the material is a microparticle with a physical lateral dimension comprised between 1 µm and 100 µm and a thickness comprised between 20 nm and 5 µm. Preferably the material is made of silicon oxide, more preferably of silicon dioxide.

A second aspect of the invention refers to a material capable of sensing ROS in a liquid media comprising a surface functionalized with an organofunctional alkylalkoxysilane molecule selected from the group consisting of aminoalkylalkoxysilane, mercaptoalkylalkoxysilane and hydroxyalkylalkoxysilane, directly linked to an activated surface of a material with hydroxyl groups, wherein the surface is further functionalized with a ROS sensor, wherein the sensor is attached to the organofunctional alkylalkoxysilane through a covalent bond, preferably through an amide bond.

In a preferred embodiment the ROS sensor is 2',7'-dichlorodihydrofluorescein diacetate, or a salt, solvate, stereoisomer or ester therefrom.

In another preferred embodiment the organofunctional alkylalkoxysilane molecule is an organofunctional aminoalkylalkoxysilane molecule selected from the group consisting of 3-aminopropyltriethoxysilane (APTES), 3-aminopropylmethyldiethoxysilane (APMDES), 3-aminopropyldimethylethoxysilane (APDMES), (3-aminopropil)trimetoxisilano (APTMS) and aminoethylaminopropyltriethoxysilane, preferably wherein the organofunctional aminoalkylalkoxysilane molecule is APTES.

In another preferred embodiment the sensor is attached to the organofunctional alkylalkoxysilane through an amide bond, wherein the ROS sensor is 2',7'-dichlorodihydrofluorescein diacetate, or a salt, solvate, stereoisomer or ester therefrom, and wherein the organofunctional alkylalkoxysilane molecule is APTES.

A third aspect of the invention refers to a material capable of sensing ROS in a liquid media obtainable by the method of the first aspect of the invention or any of its preferred embodiments.

In a preferred embodiment the surface of the material is made of silicon oxide, preferably the material is made of silicon oxide. Preferably, the silicon oxide is silicon dioxide. Other potentially useful materials include any silicon derivative, such as silicon oxide, polysilicon, monocrystalline silicon.

In another preferred embodiment the material is a microparticle with a physical lateral dimension comprised between 1 µm and 100 µm and a thickness comprised between 20 nm and 5 µm. Preferably the material is made of silicon oxide, more preferably of silicon dioxide.

A fourth aspect of the invention refers to a system comprising any of the materials of the second or third aspects of the invention or any of their preferred embodiments.

A fifth aspect of the invention refers to *in vitro* use of any of the materials of the materials of the second or third aspects of the invention or any of their preferred embodiments, or of the system of the fourth aspect of the invention, in a method for the determination of ROS in a liquid media.

In a preferred embodiment the determination of intracellular ROS is performed in a liquid media.

### DESCRIPTION OF THE EMBODIMENTS

As explained in the background art, there is a need for the development of reliable tools for the determination of ROS in liquid media.

The inventors have developed a method for obtaining a material capable of sensing ROS in a liquid media. As a proof of concept, they have obtained a material capable of sensing ROS by functionalizing planar silica microparticles (hereinafter referred to as SPAchips) with the ROS sensor is 2',7'-dichlorodihydrofluorescein diacetate **(Example 2).**

In the context of the present invention, the term "SPAchip" refers to a planar microparticle made of silicon dioxide as manufactured in Example 1 of the international patent application WO2015185782.

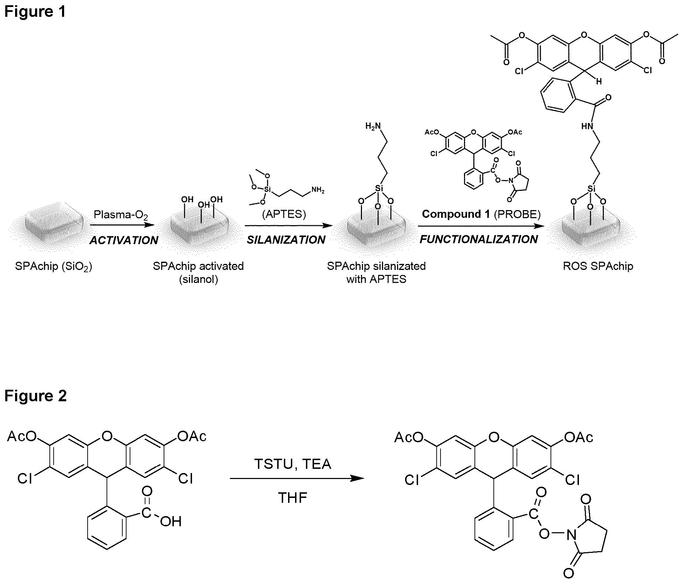

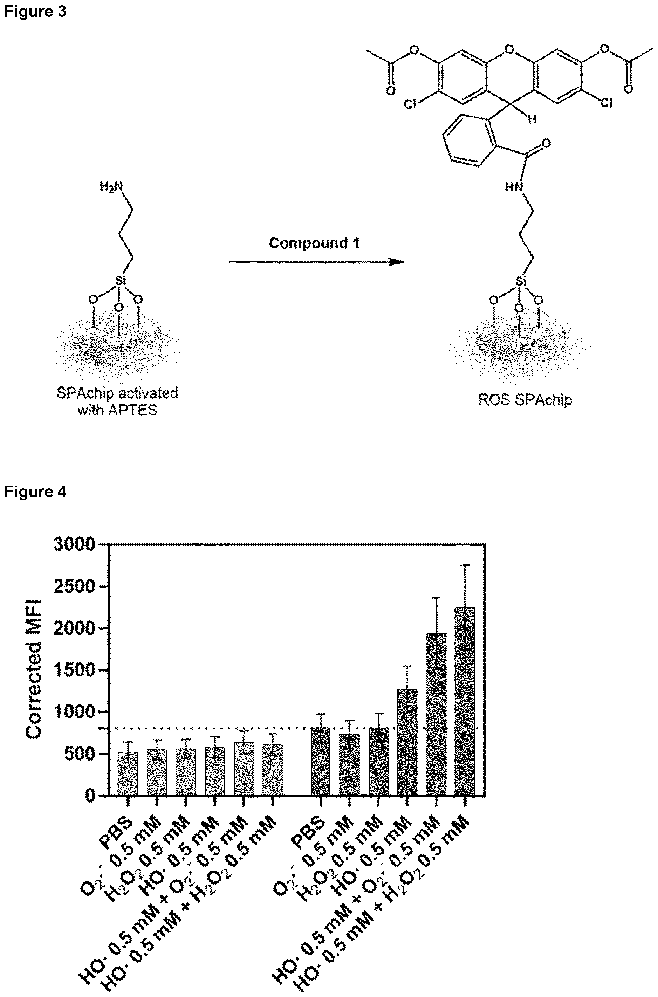

The specific process used to functionalize the microparticles is depicted in **Figure 1****.** In short, the inventors first converted the carboxyl group present at the ROS sensor (2',7'-dichlorodihydrofluorescein diacetate) into an active ester group **(****Figure 2****),** thereby generating what will be hereinafter referred to as Compound 1. Next, they activated the surface of the SPAchips. Following this, they performed a silanization of the activated surface, the hydroxyl groups, with the organofunctional molecule 3-aminopropyltriethoxysilane (APTES). Finally, they further functionalized the surface of the microparticle by reacting Compound 1 with the APTES molecule, resulting in the conjugation of both molecules through an amide bond **(****Figures 1** and **3****).**

The resulting microparticles were found to selectively detect HO• in solution **(****Figure 4****).**

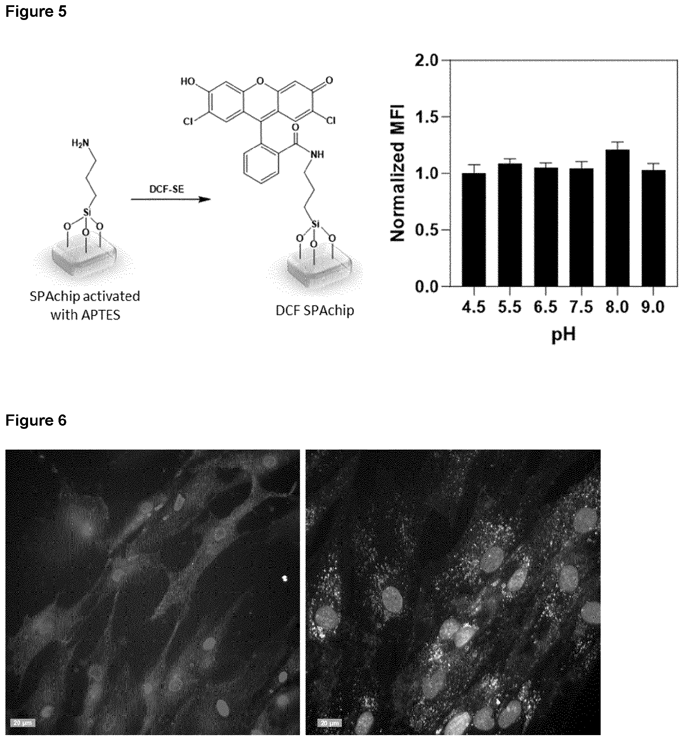

The resulting product (the functionalized microparticles) was found to present an important advantage compared to the soluble form of the 2',7'-dichlorodihydrofluorescein diacetate: while the fluorescent form of the sensor decays below pH 6, the functionalised microparticles were not sensitive to pH changes **(****Figure 5****).**

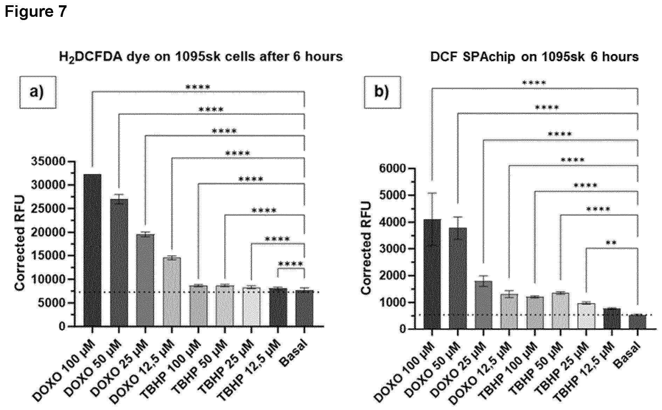

The microparticles were able to produce a fluorescent signal upon their integration in fibroblasts treated with different drugs that increase intracellular oxygen levels **(****Figure 6****).** The fluorescent signal was found to increase in a drug-dependent manner **(****Figure 7****),** thereby validating the performance of the microparticles as an intracellular ROS sensor.

Thus, the inventors herein provide a microparticle capable of sensing ROS intracellularly, as well as a method for obtaining a microparticle capable of sensing ROS intracellularly. This method provides proof of concept for the obtention of other materials capable of sensing ROS in a liquid media.

In view of the above-mentioned results, a first aspect of the invention refers to a method (hereinafter referred to as the method of the invention) for obtaining a material capable of sensing ROS in a liquid media, wherein the method comprises:
a. providing a material comprising a surface activated with hydroxyl groups,
b. carrying out a silanization process of the hydroxyl groups of step a) with an organofunctional alkylalkoxysilane molecule, and
c. reacting the silanized material resulting from step b) with a ROS sensor, wherein said reaction is under conditions suitable for the formation of a covalent bond between the ROS sensor and the organofunctional alkylalkoxysilane molecule.

Thus, step a) of the method of the present invention refers to the provision of a material comprising a surface activated with hydroxyl groups.

The term "material" refers to any material comprising an activable surface susceptible of being directly attached to an organofunctional alkylalkoxysilane molecule. Preferably, the material is any silicon derivative, such as silicon oxide, polysilicon, monocrystalline silicon, etc. More preferably, the material is or refers to SPAchips.

The term "activated with hydroxyl groups" refers to chemically modifying the surface of the material so that they expose a hydroxyl group capable of reacting with other molecules.

In a preferred embodiment the ROS is HO•.

Step b) of the method of the present invention comprises carrying out a silanization process of the hydroxyl groups of step a) with an organofunctional alkylalkoxysilane molecule.

The term "silanization" refers to the attachment of an organosilyl group to some chemical species. This conversion confers hydrophobicity to a previously hydrophilic surface. This process is often used to modify the surface properties of glass, silicon, alumina, quartz, and metal oxide substrates, which all have an abundance of hydroxyl groups.

The term "organofunctional alkylalkoxysilane molecule" refers to a type of silane compound that comprises both an alkyl and an alkoxy functional group attached to a silicon atom. These molecules are commonly used as coupling agents or surface modifiers in materials science and chemistry, especially in the synthesis of hybrid organic-inorganic materials like silane-modified polymer coatings. "Organofunctional" refers to compounds or molecules containing organic functional groups. In chemistry, "organic" refers to compounds containing carbon atoms, and "functional groups" are specific arrangements of atoms within molecules that determine their chemical properties. So, the term "organofunctional" essentially describes substances that possess both organic characteristics and specific functional groups.

In a preferred embodiment the alkylalkoxysilane molecule is selected from aminoalkylalkoxysilane, mercaptoalkylalkoxysilane, hydroxyalkylalkoxysilane and any organofunctional alkylalkoxysilane molecule useful in click chemistry.

Preferably, the alkylalkoxysilane molecule is selected from the group consisting of aminoalkylalkoxysilane, mercaptoalkylalkoxysilane and hydroxyalkylalkoxysilane.

More preferably, the organofunctional alkylalkoxysilane molecule is a (C2-C6)alkyltrialkoxysilane, preferably selected from the group consisting of an aminoalkyltrialkoxysilane such as 3-aminopropyltriethoxysilane (APTES), 3-aminopropylmethyldiethoxysilane (APMDES), 3-aminopropyldimethylethoxysilane (APDMES), (3-aminopropil)trimetoxisilano (APTMS), aminoethylaminopropyltriethoxysilane, 3-chloropropylmethyldiethoxysilane and piperazinylpropylmethyldimethoxysilane.

Most preferably, the organofunctional aminoalkylalkoxysilane molecule is APTES.

Step c) of the method of the present invention comprises reacting the silanized material resulting from step b) with a ROS sensor, wherein said reaction is under conditions suitable for the formation of a covalent bond between the ROS sensor and the organofunctional alkylalkoxysilane molecule.

The term "ROS sensor" refers to a molecular probe capable of experiencing a detectable molecular change in the presence of ROS.

It is noted that the inventors of the present invention, have used the ROS sensor 2',7'-dichlorodihydrofluorescein diacetate as a proof of concept. This molecule derives from dichlorodihydrofluorescein diacetate (H₂DCFDA), a fluorogenic dye that measures hydroxyl, peroxyl and other ROS activity within the cell. The cell-permeable H₂DCFDA diffuses into cells and is deacetylated by cellular esterases to form 2',7'-dichlorodihydrofluorescein (H₂DCF). In the presence of ROS, predominantly H₂O₂, H₂DCF is rapidly oxidized to 2',7'-dichlorofluorescein (DCF), which is highly fluorescent, with excitation and emission wavelengths of 498 and 522 nm, respectively. As mentioned above, the fluorescent form of the sensor is sensitive to pH, as it decays below pH 6, while the microparticles capable of sensing ROS of the invention are insensitive to pH fluctuations. Other ROS sensors useful in the present invention are those derived from fluorescein or rhodamine which shall also become insensitive to changes in pH once attached to the microparticle using the methodology described herein. This includes rhodamine B, rhodamine 110, rhodamine 123, fluorescein, dichlorofluorescein, among others.

Thus, in a preferred embodiment, the ROS sensor is a probe that comprises fluorescein or rhodamine.

Preferably, the ROS sensor is a probe that comprises rhodamine B, rhodamine 110, rhodamine 123, fluorescein or dichlorofluorescein, or any combination thereof.

More preferably, the probe comprises 2',7'-dichlorodihydrofluorescein diacetate, or a salt, solvate, stereoisomer or ester therefrom.

In the context of the present invention, the term "salt" must be understood as any form of a compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions.

In the context of the present invention, the term "solvate" should be understood as meaning any form of a compound in accordance with the invention in which said compound is bonded by a non-covalent bond to another molecule (normally a polar solvent), including especially hydrates and alcoholates, like for example, methanolate.

In the context of the present invention, the term "stereoisomer" should be understood as a type of isomer in which the atoms are connected in the same order, but their spatial arrangement differs. This difference in spatial arrangement can occur due to the presence of double bonds, rings or chiral centres in the molecule. Stereoisomers can be further classified in two main types:
- Conformational isomers: stereoisomers that can be interconverted by rotating around single bonds. They have the same connectivity but differ in the spatial orientation of their atoms.
- Configurational isomers: stereoisomers that cannot be interconverted without breaking bonds.

In the context of the present invention, the term "ester" should be understood as a chemical compound derived from an acid (organic or inorganic) in which at least one -OH (hydroxyl group) is replaced by an -O-alkyl (alkoxy) group. This definition includes activated esters such as the one of *Formula I* below.

Most preferably, the probe comprises a molecule of *Formula I.*

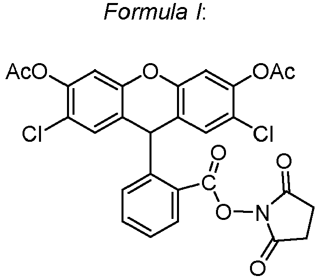

In a preferred embodiment, the surface of the material is made of silicon oxide, preferably the material is made of silicon oxide. Preferably, the silicon oxide is silicon dioxide. Other potentially useful materials include any silicon derivative, such as silicon oxide, polysilicon, monocrystalline silicon.

In a preferred embodiment, the material is a microparticle with a physical lateral dimension comprised between 1 µm and 100 µm and a thickness comprised between 20 nm and 5 µm. Preferably the material is made of silicon oxide, more preferably of silicon dioxide.

In a preferred embodiment, the liquid media is the cytoplasm.

Each embodiment disclosed herein is contemplated as being applicable to each of the other disclosed embodiments. Thus, all combinations of the various elements described herein are within the scope of the invention.

A second aspect of the invention refers to a material method capable of sensing ROS in a liquid media comprising a surface functionalized with an organofunctional alkylalkoxysilane molecule selected from the group consisting of aminoalkylalkoxysilane, mercaptoalkylalkoxysilane and hydroxyalkylalkoxysilane, directly linked to an activated surface of a material with hydroxyl groups, wherein the surface is further functionalized with a ROS sensor, wherein the sensor is attached to the organofunctional alkylalkoxysilane through a covalent bond, preferably through an amide bond.

In a preferred embodiment the ROS is HO•.

The organofunctional alkylalkoxysilane molecules are the same as described for the first aspect. Thus, all the embodiments of the description mentioned in this regard for the first aspect applies herein.

The ROS sensors are the same as described for the first aspect. Thus, all the embodiments of the description mentioned in this regard for the first aspect applies herein.

In a preferred embodiment, the liquid media is the cytoplasm.

A third aspect of the invention refers to a material capable of sensing ROS in a liquid media obtainable by any of the methods of the first embodiment of the invention.

In a preferred embodiment the ROS is HO•.

In a preferred embodiment, the surface of the material is made of silicon oxide, preferably the material is made of silicon oxide. Preferably, the silicon oxide is silicon dioxide. Other potentially useful materials include any silicon derivative, such as silicon oxide, polysilicon, monocrystalline silicon.

In a preferred embodiment, the material is a microparticle with a physical lateral dimension comprised between 1 µm and 100 µm and a thickness comprised between 20 nm and 5 µm. Preferably the material is made of silicon oxide, more preferably of silicon dioxide.

In a preferred embodiment, the liquid media is the cytoplasm.

A fourth aspect of the invention refers to a system comprising the material of any of the materials of the second and third embodiments of the invention.

The term "system", in the context of the present invention, refers to a device that comprises a surface capable of bearing any of the materials of the invention, or a device made of the material of the invention and that it is intended for use in the determination of ROS in a specific liquid medium. This system may be a macroscopic device intended for determining ROS in a liquid medium, such as a reactor wherein a part of the surface is covered by any of the materials of the invention intended for use in the determination of ROS in a liquid medium (such as in a cell culture medium), or a microarray wherein a portion of the surface is covered by any of the material of the invention intended for use in the determination of ROS in a liquid medium (such as in a biological sample), among others. It may also be a nanoscopic or microscopic device, such as a nanoparticle or a microparticle covered by any of the materials of the invention intended for use in the determination of ROS intracellularly. The nature of the system is determined by the nature of the liquid medium in which one intends to perform the determination of ROS.

In a preferred embodiment the ROS is HO•.

A fifth aspect of the invention refers to the *in vitro* use of any of the materials of the second or third aspects of the invention or any of their preferred embodiments, or of the system of the fourth embodiment of the invention or any of its preferred embodiments, in a method for the determination of ROS in a liquid media.

The term *"in vitro* use" has been used with the intention of excluding the use *in vivo* in an animal, including humans. It does not exclude however the use in unicellular organisms (such as bacteria) or in *in vitro* eukaryotic models (such as animal or human cell lines and tissue-derived organoids).

In a preferred embodiment the ROS is HO•.

In a preferred embodiment, the determination of intracellular ROS in a liquid media.

Terms not specifically defined herein should be given the meanings that would be given to them by one of skill in the art in light of the disclosure and the context. As used in the specification, however, unless specified to the contrary, the following terms have the meaning indicated and the following conventions are adhered to.

Throughout the present specification and the accompanying clauses, the words "comprise" and variations such as "comprises", "comprising" are to be interpreted inclusively. That is, these words are intended to convey the possible inclusion of other elements or integers not specifically recited, where the context allows. The word "comprise" also includes the term "consists of".

For the purposes of the present invention, any ranges given include both the lower and the upper end-points of the range.

### EXAMPLES

The present invention is illustrated by means of the Examples set below without the intention of limiting its scope of protection.

### Example 1. Material and Methods

Nitric Acid (HNO3) 69%, (3-aminopropyl) triethoxysilane (APTES), Dimethyl sulfoxide (DMSO) 99%, Sodium bicarbonate (NaHCO3), Phosphate buffered saline (PBS) pH 7.4, Iron (II) Sulfate Heptahydrate (FeSO4·7H2O), 30% Hydrogen Peroxide solution (H2O2), Fluoromount^{™} aqueous mounting medium, Triethylamine (TEA), Tetrahydrofuran (THF), Ethanol absolute and Tetramethyl-O-(N-succinimidyl)uronium tetrafluoroborate (TSTU) were obtained from Merck. D-Sorbitol and 2',7'-dichlorodihydrofluorescein (H2DCFDA) was obtained from Fisher Scientific. Ethanol 96%, 2-Propanol, Hexane and Ethyl Acetate were obtained from Scharlab. Sylgard and Silicone Elastomer Curing Agent were obtained from Farnell Components. Dimethylsiloxane (60-70% ethylene oxide) Block copolymer (DBE) was obtained from Cymitquimica.

Equipment: Mili-Q IQ7003 Ultrapure and Pure Water Purification System (Merck), High power expanded plasma cleaner (PDC-002-CE) (Harrick Plasma), Dry Heat Sterilizer 55 LT (FN 055) (Nüve), Spectrafuge mini-centrifuge 24D (LabNet), Leica DM4 B Upright Microscope (Leica Microsystems), Operetta CLS (PerkinElmer)

Dulbecco's Modified Eagle medium (DMEM, high glucose w/o L-glutamine and w/o phenol red, Gibco^{™}), trypsin-EDTA (0.25%), Invitrogen^{™} CellMask^{™} deep red and Hoechst 3343 solution (20 mM) were purchased from ThermoScientific^{™}. Tert-butyl 6-aminocaproate (TBHP, 97%) was acquired from CymitQuimica. Phosphate buffered saline modified without calcium chloride and without magnesium chloride (PBS 1x, pH 7.4), fetal bovine serum (FBS, for standard applications), doxorubicin hydrochloride (DOXO), L-glutamine (200mM), penicillin-streptomycin solution (100x) and dimethyl sulfoxide (DMSO) were purchased from Sigma-Merck.

### Example 2. Procedures

The manufacture of ROS SPAchips was performed using the following procedures (illustrated in **Figure 1****):** wafer manufacture, activation, silanization, chemical probe-synthesis and functionalization.

### Wafer Manufacturing

SPAchip wafers were manufactured by D+T Microelectrónica (Barcelona, Spain) according to the methodology protected by the patent family EP3153855B1 and described previously. Briefly, a 1 µm thick thermal SiO2 layer was grown by wet oxidation on both sides over a diameter of 100 mm and 300 µm thick P-type silicon wafers. Positive photoresist was spun onto the wafers and then, exposure to UV light was performed through a photomask, to define the shape and lateral dimensions of the chips and baked. Then, dry-etching with C2F6 and CHF3 mixture was applied to engrave the chips and the photoresist was stripped by plasma etching. Finally, feet underneath each chip were defined by Reactive Ion Etching (RIO) with SF6 and C4F8 atmosphere.

### Activation/Silanization

Amine-terminated self-assembled monolayers (SAMs) were prepared as linkers to bind fluorophores onto the chips. To that aim, wafer surfaces were activated by plasma, after this step, the wafers were immersed in a 30% nitric acid solution for 25 minutes at 75°C. Subsequently, the wafers were rinsed with Milli-Q water and dried using N2(g). Then, immersed in an ethanolic solution of APTES. After curing (110°C, 1 hour), silanized wafers were rinsed in ethanol and Mili-Q water prior to fluorophore attachment.

### Synthesis of compound 1 (chemical probe)

A solution of H₂DCFDA (60 mg, 0.12 mmol) in THF (1.2 mL) was treated, under N₂ atmosphere, with TEA (26 mL, 0.19 mmol) and TSTU (56 mg, 0.19 mmol). This step is illustrated in **Figure 2****.** The reaction wall allowed to stir 24 h at room temperature, and solvent were evaporated in vacuo. The crude product was purified by flash silica gel chromatography (Hexane/ethyl acetate 8:2 to 65:35) to yield the desired product as a white solid (69 g, 96%). ¹H NMR (300 MHz, CDCl3) δ 8.06 (dd, J= 7.8, 1.4 Hz, 1H), 7.50 (td, J= 7.7, 1.4 Hz, 1H, Ar), 7.36 (td, J=7.7, 1.2 Hz, 1H, Ar), 7.16 (s, 2H, Ar), 7.14 (br.s, 1H, Ar), 6.95 (s, 2H, Ar), 6.18 (s, 1H, CH), 2.95 (s, 4H, 2CH3), 2.33 (s, 6H, 2OAc).

### SPAchip functionalization with Compound 1 (ROS SPAchip)

This step is illustrated in **Figure 3****.** Fluorophore printing stock solution with Compound 1 (depicted in **Figure 1**) was prepared in DMSO and diluted in sodium bicarbonate buffer 10 mM pH 9.0 to print on SPAchips. Poly di-methyl siloxane (PDMS) stamps were prepared by mixing Sylgard, Silicone Elastomer Curing Agent and DBE on culture dishes and curing it at 75°C for 1 hour. PDMS stamps were activated with oxygen plasma and inked with 10 uL/cm² of compound 1 solution and then applied on silanized SPAchip wafers for 30 minutes at room temperature. Excess of fluorophores was rinsed with 2-Propanol and Mili-Q water.

### Release and collection of functionalized SPAchip

A drop of Fluoromount^{™} mounting medium was placed on top of the surface of the functionalized SPAchip wafers and left to solidify in a Dry Heat Sterilizer at 75°C for 1 hour. A lateral force was used to separate the solidified medium from the substrate, carrying the released chip with it and collected in individual sterile 1.5 mL tubes.

The solidified medium was dissolved in a buffer solution (PBS solution containing Sorbitol) and centrifuged at 8000 rpm for 10 minutes, the supernatant was aspirated and discarded, the buffer solution was added back into the tube and the above steps were repeated. Finally, the pellet was resuspended in the buffer solution.

### Data acquisition and statistical analysis

Fluorescence images of control and test items were taken with a High Content Screening microscope (Operetta, Perkin Elmer). Within each set of experiments, all images were taken with the same acquisition settings for H₂DCFDA to minimize experimental variability. When possible, settings were fine-tuned for reducing phototoxicity and photobleaching, minimizing electronic noise, and to avoid saturate pixels. Image analysis was performed with Columbus software.

Selected z-stack was integrated in an image by applying a "Maximum intensity z-projection" algorithm. Different regions of interest (ROI) were defined per image, depending on the analysis purpose, and the following fluorescence parameters were measured: Fluorescence mean, standard deviation, area, mode, minimum, maximum, corrected intensity and background intensity mean. Raw data were copy-pasted and saved in Excel files. Each set of samples was analysed with the same settings.

All the collected data were analysed with GraphPad Prism 10. ROUT method was used for identifying outliers. For statistical analysis, alpha value was set to 0.05 (95% confidence interval). For analysing the response to OH· changes, fluorescence signal was plotted vs OH· concentrations. Linear regressions were made, and slopes and regressions compared among them.

### Cell culture experiments

Fibroblast human cells CCD-1095sk (BioNova 300642) was employed during this work by cultured with DMEM high glucose. Cell culture medium was supplemented with 10% FBS, 2 mM of L-glutamine and 1% streptomycin-penicillin to obtain complete medium. All the cell cultures were incubated at 37°C in a Nuve EC160 incubator (5% CO₂). All the procedures were performed inside a laminar flow hood BiOptima 5 (Telstar).

After that, 3000 cells/well were culture in a PhenoPlate 384-well black, optically clear flat-bottom (PerkinElmer) in its complete medium. Fibroblast were then incubated overnight into a cell incubator at 37°C and CO₂ 5% to allow 50-70% confluency. After 24 hours, they were treated with the corresponding concentration of SPAchip resuspended in DMEM without Phenol Red for 24 hours.

Once the SPAchips have been internalized in the cells, nuclear and cytoplasmatic stain is added. Hoechst 3342 is used as nuclear live cell stain and Cell Mask deep red is added to stain the cytoplasmatic environment. Both are incubated in the cells for 30 minutes and then they are discarded by washing with PBS 1x.

The next step consists into design an experimental template where located the selected treatments as well as the control wells to check the stability of the SPAchips. First, basal measurements were prepared with cell culture as DMEM without phenol red for negative control. In addition, DOXO and TBHP treatments diluted in the appropriate fresh culture medium at the appropriate concentrations (100, 50, 25 and 12.5 µM) for positive control. Measurements starts after 6 hours of treatment by using Perkin Elmer Operetta with 37°C and 5% CO₂ using a water immersion objective at a 40x magnification to identify the intracellular and extracellular SPAchips.

### Example 3. Results

### Example 3.1. ROS SPAchips response in solution

The selectivity of compound 1 to different ROS was studied. For this study, 80 µL per well of different measured in the Operetta (λex: 460 - 490 nm; λem: 500 - 550 nm) **(****Figure 4****).**

The fluorescence intensity increases with time for SPAchips dispersed in the ROS solutions for periods of more than 96h **(****Figure 4****).** ROS SPAchips are selective for HO•. The response observed in mixtures HO• + H2O2 and HO• + O2•- is due to the generation of more HO• through Fenton and Haber-Weiss reactions.

The fluorescence of soluble form of H₂DCFDA, commonly used for ROS detection in cells, decays below pH 6. On the other hand, one of the additional advantages of ROS SPAchips is that they are not sensitive to pH changes even in their oxidized form: DCF SPAchips **(****Figure 5****).**

### Example 3.2. ROS SPAchips on cells

Treated fibroblast cells with DOXO and TBHP for 6 hours were observed by High Content Screening to detect the SPAchip fluorescence signal. Their signal is dependent on the ROS amount produced by the cells due to each drug concentration **(****Figure 6****).**

In addition to the cellular characterization by HCS, the intensity fluorescence signal from the SPAchips were collected to obtain the intracellular ROS variation depending on the drug concentration. Furthermore, those signals were compared to a commercial ROS dye as H₂DCFDA that was incubated before the drug treatments. SPAchip fluorescence signals shown an increment in the intensity as a function of the drug concentration **(****Figure 7****).**

## Claims

1. A method for obtaining a material capable of sensing reactive oxygen species in a liquid media, wherein the method comprises:
a. providing a material comprising a surface activated with hydroxyl groups,
b. carrying out a silanization process of the hydroxyl groups of step a) with an organofunctional alkylalkoxysilane molecule, selected from the group consisting of aminoalkylalkoxysilane, mercaptoalkylalkoxysilane and hydroxyalkylalkoxysilane, and
c. reacting the silanized material resulting from step b) with a reactive oxygen species sensor, wherein said reaction is under conditions suitable for the formation of a covalent bond between the reactive oxygen species sensor and the organofunctional alkylalkoxysilane molecule.

2. The method, according to claim 1, wherein the reactive oxygen species sensor is 2',7'-dichlorodihydrofluorescein diacetate, or a salt, solvate, stereoisomer or ester therefrom.

3. The method, according to any of the claims 1 or 2, wherein step c) comprises reacting the silanized material resulting from step b) with the reactive oxygen species sensor 2',7'-dichlorodihydrofluorescein diacetate, wherein the organofunctional alkylalkoxysilane molecule is an organofunctional aminoalkylalkoxysilane molecule, wherein said reaction is under conditions suitable for the formation of an amide bond between the carboxylic group present at the reactive oxygen species sensor 2',7'-dichlorodihydrofluorescein diacetate, and an amine group present at the organofunctional aminoalkylalkoxysilane molecule.

4. The method, according to claim 3, wherein the organofunctional aminoalkylalkoxysilane molecule is selected from the group consisting of 3-aminopropyltriethoxysilane (APTES), 3-aminopropylmethyldiethoxysilane (APMDES), 3-aminopropyldimethylethoxysilane (APDMES), (3-aminopropil)trimetoxisilano (APTMS) and aminoethylaminopropyltriethoxysilane.

5. The method, according to any of the claims 3 or 4, wherein the organofunctional aminoalkylalkoxysilane molecule is APTES.

6. The method, according to any of the claims 3 to 5, wherein the reactive oxygen species sensor is 2',7'-dichlorodihydrofluorescein diacetate, or a salt, solvate, stereoisomer or ester therefrom, and wherein the organofunctional aminoalkylalkoxysilane molecule is APTES.

7. The method, according to any of the claims 1 to 6, wherein the surface of the material is made of silicon oxide, preferably wherein the material is made of silicon oxide.

8. The method, according to any of the claims 1 to 7, wherein the material is a microparticle with a physical lateral dimension comprised between 1 µm and 100 µm and a thickness comprised between 20 nm and 5 µm.

9. A material capable of sensing reactive oxygen species in a liquid media comprising a surface functionalized with an organofunctional alkylalkoxysilane molecule selected from the group consisting of aminoalkylalkoxysilane, mercaptoalkylalkoxysilane and hydroxyalkylalkoxysilane, directly linked to an activated surface of a material with hydroxyl groups, wherein the surface is further functionalized with a reactive oxygen species sensor, wherein the sensor is attached to the organofunctional alkylalkoxysilane through a covalent bond, preferably through an amide bond.

10. The material, according to claim 9, wherein the reactive oxygen species sensor is 2',7'-dichlorodihydrofluorescein diacetate, or a salt, solvate, stereoisomer or ester therefrom.

11. The material, according to any of the claims 9 or 10, wherein the organofunctional alkylalkoxysilane molecule is an organofunctional aminoalkylalkoxysilane molecule selected from the group consisting of 3-aminopropyltriethoxysilane (APTES), 3-aminopropylmethyldiethoxysilane (APMDES), 3-aminopropyldimethylethoxysilane (APDMES), (3-aminopropil)trimetoxisilano (APTMS) and aminoethylaminopropyltriethoxysilane, preferably wherein the organofunctional aminoalkylalkoxysilane molecule is APTES.

12. The material, according to any of the claims 9 to 11, wherein the sensor is attached to the organofunctional alkylalkoxysilane through an amide bond, wherein the reactive oxygen species sensor is 2',7'-dichlorodihydrofluorescein diacetate, or a salt, solvate, stereoisomer or ester therefrom, and wherein the organofunctional alkylalkoxysilane molecule is APTES.

13. A material capable of sensing reactive oxygen species in a liquid media obtainable by any of the methods of claims 1 to 8.

14. The material, according to any of the claims 9 to 13, wherein the surface of the material is made of silicon oxide, preferably wherein the material is made of silicon oxide.

15. The material, according to any of the claims 9 to 14, wherein the material is a microparticle with a physical lateral dimension comprised between 1 µm and 100 µm and a thickness comprised between 20 nm and 5 µm.

16. System comprising the material of any of the claims 9 to 15.

17. *In vitro* use of any of the materials of claims 9 to 15, or of the system of claim 16, in a method for the determination of reactive oxygen species in a liquid media.

18. *In vitro* use, according to claim 17, in a method for the determination of intracellular reactive oxygen species in a liquid media.
